# EUROPEAN PATENT APPLICATION

(11) **EP 4 434 536 A1**
(43) Date of publication of application: **25.09.2024**
(21) Application number: 22894677.8
(22) Date of filing: 08.11.2022
(51) Int. Cl.: A61K 38/26, C07K 14/605, A61P 3/10

(54) **STAPLED PEPTIDE AND USE THEREOF**

(30) Priority: 19.11.2021 CN 202111398470; 09.12.2021 CN 202111500873
(71) Applicant: Medshine Discovery Inc., Nanjing, Jiangsu 210032 (CN)
(72) Inventor: PAN, Zhixiang, Shanghai 200131 (CN); HE, Haiying, Shanghai 200131 (CN); JIANG, Zhigan, Shanghai 200131 (CN); CHEN, Shuhui, Shanghai 200131 (CN)
(74) Representative: Dehns
(86) International application number: PCT/CN2022/130705
(87) International publication number: WO 2023/088140

(57) **Abstract**

A series of stapled peptides and a use thereof, specifically relating to a polypeptide having a sequence as shown in a formulas (I-1)-(I-5) and (II-6), and a pharmaceutically acceptable salt thereof.

## Description

### REFERENCES TO RELATED APPLICATIONS

The present application claims priorities and benefits of Chinese invention patent application No. 202111398470.4 filed to China National Intellectual Property Administration on November 19, 2021, and Chinese invention patent application No. 202111500873.5 filed to China National Intellectual Property Administration on December 9, 2021, and the contents disclosed in the applications are incorporated herein by reference in their entireties.

### TECHNICAL FIELD

The present disclosure relates to a series of stapled peptides and a use thereof, specifically to a polypeptide having a sequence as shown in formulas (I-1)-(I-5) or (II-6), and a pharmaceutically acceptable salt thereof.

### BACKGROUND

Diabetes is a group of metabolic diseases characterized by hyperglycemia, which is caused by defect in insulin secretion, or impairment of its biological function, or by both reasons. Long-term hyperglycemia can lead to chronic damage and functional disorders of various tissues, especially eyes, kidneys, heart, blood vessels, and nerves. The latest data from the International Diabetes Federation in 2019 shows that approximately 460 million people worldwide suffer from diabetes. In 2020, there are nearly 130 million diabetic patients in China, therefore China has become the country with the largest number of diabetic patients in the world. According to a national cross-sectional study in China published in the British Medical Journal in 2020, the national prevalence of type 2 diabetes (T2D) is 11.2%, while only 49.4% of which reach the target of glycated hemoglobin. T2D is a progressive disease with a very high risk of complications such as diabetic nephropathy, diabetic retinopathy, and diabetic foot at the advance stages of the disease. It can be seen that T2D is a major health challenge for humanity.

Glucose-dependent insulinotropic polypeptide (GIP) and glucagon-like peptide-1 (GLP-1) both belong to incretins, and they can promote the release of insulin and are glucose-dependent, thus the risk of causing hypoglycemia is low. GLP-1 has the effect of suppressing appetite in the brain, delaying gastric emptying, reducing hepatic gluconeogenesis, and increasing glucose consumption in muscle tissue; GIP has the effect of increasing glucose consumption in adipose tissue, promoting lipid metabolism, and preventing ectopic fat accumulation; Glucagon (GCG) is secreted by pancreatic α cells, and has the effect of inhibiting hepatic fat synthesis decreasing and cholesterol levels, increasing the fat burning rate of brown adipose tissue, and protecting myocardial cells and cardiac function. Therefore, the triple activation of GIPR, GLP-1R, and GCGR will have a synergistic effect on the regulation of glucose/lipid metabolism, and may play a more positive role in lowering blood sugar, reducing weight, alleviating fatty liver, and cardiovascular protection.

### CONTENT OF THE PRESENT INVENTION

The present disclosure provides a polypeptide with a sequence shown in the following formula or a pharmaceutically acceptable salt thereof,

Tyr-Aib-Gln-Gly-Thr-Phe-Thr-Ser-Asp-Tyr-Ser-Ile-X₁-Leu-Asp-Lys-¹Lys-Ala-Gln-¹Lys-Ala-Phe-Ile-Glu-Tyr-Leu-Leu-Glu-Gly-Gly-Pro-Ser-Ser-Gly-Ala-Pro-Pro-Pro-X₂ (I-1),

Tyr-Aib-Gln-Gly-Thr-Phe-Thr-Ser-Asp-Tyr-Ser-Ile-X₁-Leu-Asp-¹Lys-Lys-Ala-¹Lys-Aib-Ala-Phe-Ile-Glu-Tyr-Leu-Leu-Glu-Gly-Gly-Pro-Ser-Ser-Gly-Ala-Pro-Pro-Pro-X₂ (I-2),

Tyr-Aib-Gln-Gly-Thr-Phe-Thr-Ser-Asp-Tyr-Ser-Ile-X₁-Leu-Asp-Lys-Lys-Ala-Gln-¹Lys-Ala-Phe-¹Lys-Glu-Tyr-Leu-Leu-Glu-Gly-Gly-Pro-Ser-Ser-Gly-Ala-Pro-Pro-Pro-X₂ (I-3),

Tyr-Aib-Gln-Gly-Thr-Phe-Thr-Ser-Asp-Tyr-Ser-Ile-Xi-Leu-Asp-Lys-Lys-Ala-Gln-Aib-¹Lys-Phe-Ile-¹Lys-Tyr-Leu-Leu-Glu-Gly-Gly-Pro-Ser-Ser-Gly-Ala-Pro-Pro-Pro-X₂ (I-4),

Tyr-Aib-Gln-Gly-Thr-Phe-Thr-Ser-Asp-Tyr-Ser-Ile-Xi-Leu-Asp-Lys-Lys-Ala-Gln-Aib-Ala-Phe-Ile-Glu-¹Lys-Leu-Leu-¹Lys-Gly-Gly-Pro-Ser-Ser-Gly-Ala-Pro-Pro-Pro-X₂ (I-5),

Tyr-Aib-Gln-Gly-Thr-Phe-Thr-Ser-Asp-Tyr-Ser-Ile-Xi-Leu-Asp-Lys-Lys-Ala-Gln-Aib-Ala-Phe-Ile-¹Lys-Tyr-Leu-¹Lys-Glu-Gly-Gly-Pro-Ser-Ser-Gly-Ala-Pro-Pro-Pro-X₂ (II-6),
wherein
Aib is 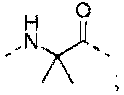
X₁ is 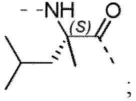
X₂ is selected from 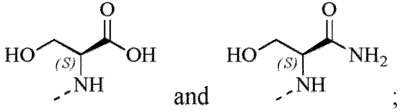
¹Lys represents a lysine with a modification, wherein the modification is that amino groups on side chains of two lysines are connected to 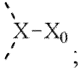
X is selected from 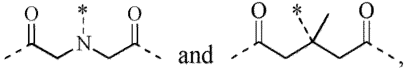 wherein "*" indicates a position connected to X₀;
X₀ is 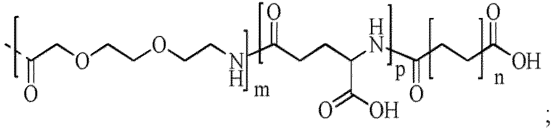
m is selected from 2 and 3;
n is selected from 8, 9, and 10;
p is selected from 1 and 2.

In some embodiments of the present disclosure, the m is 2, and other variables are as defined in the present disclosure.

In some embodiments of the present disclosure, the n is 9, and other variables are as defined in the present disclosure.

In some embodiments of the present disclosure, the p is 1, and other variables are as defined in the present disclosure.

In some embodiments of the present disclosure, the X₂ is 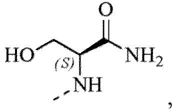 and other variables are as defined in the present disclosure.

In some embodiments of the present disclosure, the X₀ is 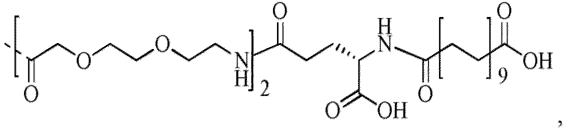 and other variables are as defined in the present disclosure.

In some embodiments of the present disclosure, the 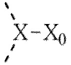 is 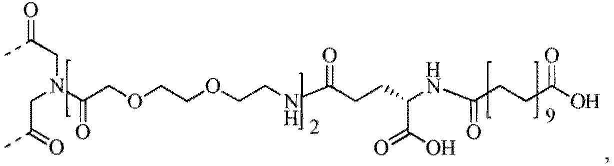 and other variables are as defined in the present disclosure.

The present disclosure also provides a polypeptide shown in the following formula or a pharmaceutically acceptable salt thereof,

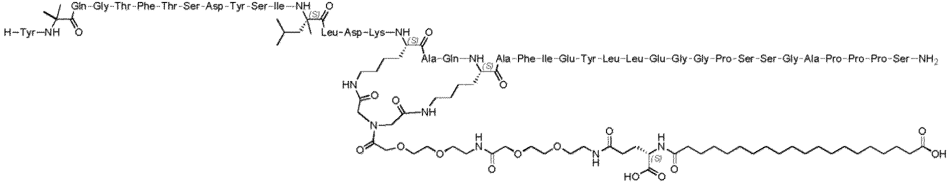

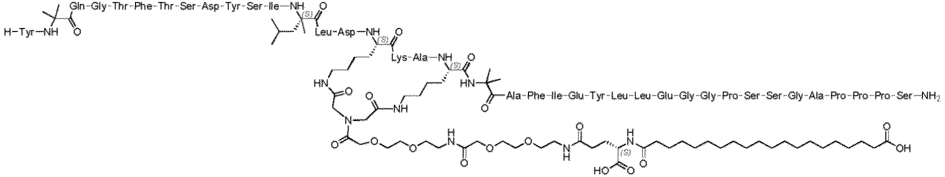

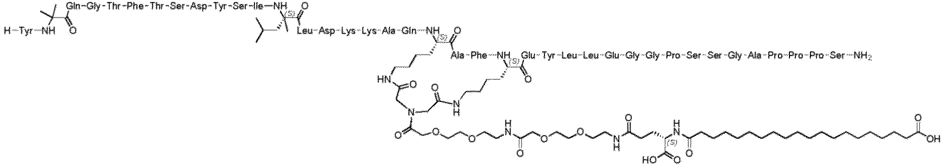

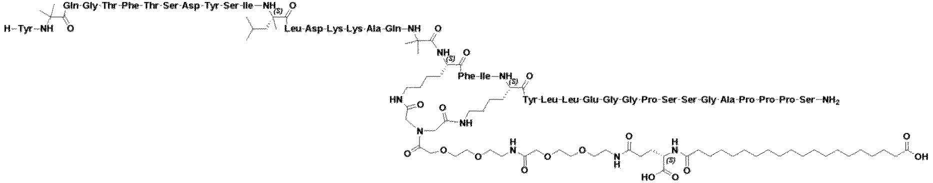

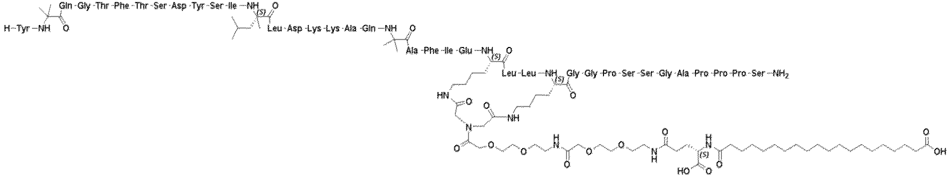

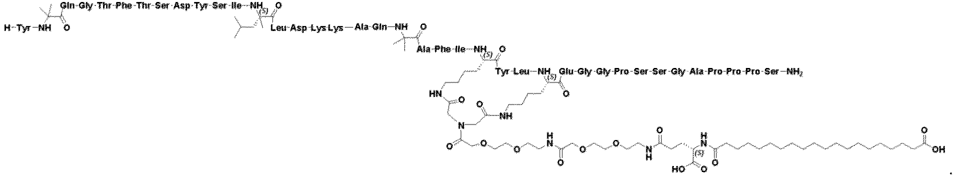

The present disclosure also provides a pharmaceutical composition comprising a therapeutically effective amount of the polypeptide as an active ingredient or the pharmaceutically acceptable salt thereof, and a pharmaceutically acceptable carrier.

In some embodiments of the present disclosure, a use of the polypeptide or the pharmaceutically acceptable salt thereof, or the pharmaceutical composition in the preparation of a medicament for treating diabetes and/or obesity is provided.

### Technical effect

The polypeptide of the present disclosure has strong agonistic activity on GLP-1R/GIPR; it has excellent pharmacokinetic properties in multiple species and exhibits excellent weight loss efficacy in DIO mice and excellent hypoglycemic effects in db/db mice.

### Definition and description

Unless otherwise specified, the following terms and phrases when used herein have the following meanings. A specific term or phrase should not be considered indefinite or unclear in the absence of a particular definition, but should be understood in the ordinary meanings. When a trading name appears herein, it is intended to refer to its corresponding commodity or active ingredient thereof.

The term "pharmaceutically acceptable" is used herein in terms of those polypeptides, materials, compositions, and/or dosage forms, which are suitable for use in contact with human and animal tissues within the scope of reliable medical judgment, without inducing excessive toxicity, irritation, an allergic reaction, or other problems or complications, and are commensurate with a reasonable benefit/risk ratio.

The term "pharmaceutically acceptable salt" refers to a salt of the polypeptide of the present disclosure that is prepared by the polypeptide having a specific substituent of the present disclosure with a relatively non-toxic acid or base. When the polypeptide of the present disclosure has a relatively acidic functional group, a base addition salt can be obtained by bringing such polypeptide into contact with a sufficient amount of base in a pure solution or a suitable inert solvent. Certain specific polypeptides of the present disclosure have both basic and acidic functional groups, thus can be converted to any base or acid addition salt.

The pharmaceutically acceptable salt of the present disclosure can be synthesized by conventional chemical methods from parent polypeptides having acid radicals or bases. Generally, such salt can be prepared by reacting the free acid or base form of these polypeptides with a stoichiometric amount of an appropriate base or acid in water or an organic solvent or a mixture of both.

"Amino acid" refers to naturally occurring and synthetic amino acids, as well as amino acid analogs and amino acid mimetics that function like naturally occurring amino acids. Naturally occurring amino acids are those encoded by the genetic code, as well as those that are later modified, such as hydroxyproline, γ-carboxyglutamic acid, and O-phosphoserine. Amino acid analogs refer to polypeptides that have the same basic chemical structure (e.g., α-carbon bound to hydrogen, carboxyl group, amino group, and R group) as naturally occurring amino acids, such as homoserine, norleucine, methionine sulfoxide, methionine methylsulfonium. Such analogs may have modified R groups (e.g., norleucine) or modified peptide backbones, but retain the same basic chemical structures as the naturally occurring amino acids. Amino acid mimetic refers to a chemical compound whose structure is different from the general chemical structure of amino acids but plays a similar role to naturally occurring amino acids.

A or Ala described herein represents alanine, which has a structure of 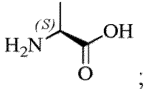 R or Arg represents arginine, which has a structure of 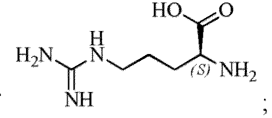 N or Asn represents asparagine, which has a structure of 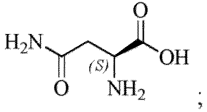 D or Asp represents aspartic acid, which has a structure of 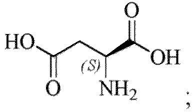 C or Cys represents cysteine, which has a structure of 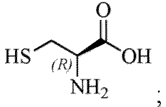 Q or Gln represents glutamine, which has a structure of 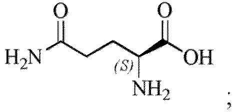 E or Glu represents glutamic acid, which has a structure of 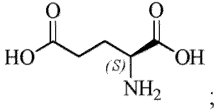 G or Gly represents glycine, which has a structure of 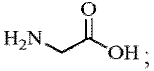 H or His represents histidine, which has a structure of 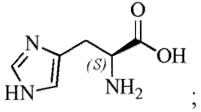 I or Ile represents isoleucine, which has a structure of 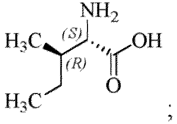 L or Leu represents leucine, which has a structure of 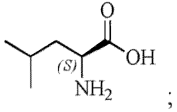 K or Lys represents lysine, which has a structure of 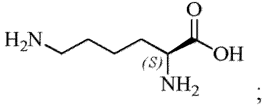 M or Met represents methionine, which has a structure of 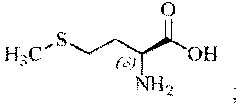 F or Phe represents phenylalanine, which has a structure of 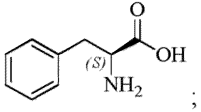 P or Pro represents proline, which has a structure of 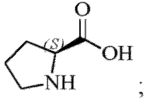 S or Ser represents serine, which has a structure of 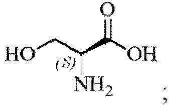 T or Thr represents threonine, which has a structure of 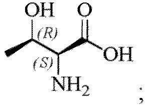 W or Trp represents tryptophan, which has a structure of 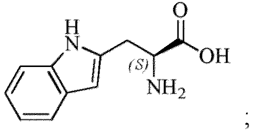 Y or Tyr represents tyrosine, which has a structure of 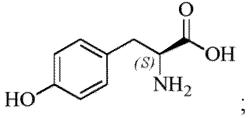 V or Val represents valine, which has a structure of

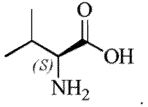

The term "treatment" includes inhibiting, slowing, stopping or reversing the progression or severity of existing symptoms or illnesses.

Unless otherwise specified, the term "isomer" is intended to include a geometric isomer, a cis-trans isomer, a stereoisomer, an enantiomer, an optical isomer, a diastereoisomer, and a tautomeric isomer.

The polypeptides of the present disclosure may exist in specific geometric or stereoisomeric forms. The present disclosure contemplates all such polypeptides, including *cis* and *trans* isomers, (-)- and (+)-enantiomers, (*R*)- and (*S*)-enantiomers, diastereoisomers, (*D*)-isomers, (*L*)-isomers, racemic mixtures, and other mixtures thereof, such as enriched mixtures of enantiomer or diastereomer, all of which fall within the scope of the present disclosure. Additional asymmetric carbon atoms may be present in substituents such as alkyl. All these isomers and their mixtures are encompassed within the scope of the present disclosure.

Unless otherwise specified, the term "enantiomer" or "optical isomer" refers to stereoisomers that are mirror-image.

Unless otherwise specified, the term "*cis-trans* isomer" or "geometric isomer" is caused by the inability to rotate freely of double bonds or single bonds of ring-forming carbon atoms.

Unless otherwise specified, the term "diastereomer" refers to a stereoisomer in which a molecule has two or more chiral centers and the molecules are non-mirror images.

Unless otherwise specified, "(+)" refers to dextrorotation, "(-)" refers to levorotation, and "(±)" refers to racemic.

Unless otherwise specified, the absolute configuration of a stereogenic center is represented by a wedged solid bond ( 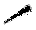 ) and a wedged dashed bond ( 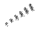 ), and the relative configuration of a stereogenic center is represented by a straight solid bond ( 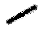 ) and a straight dashed bond ( 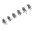 ), a wave line ( 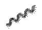 ) is used to represent a wedged solid bond ( 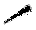 ) or a wedged dashed bond ( 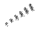 ), or the wave line ( 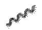 ) is used to represent a straight solid bond ( 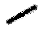 )or a straight dashed bond ( 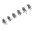 ).

Unless otherwise specified, the terms "enriched in one isomer", "enriched in isomers", "enriched in one enantiomer", or "enriched in enantiomers" refer to the content of one of the isomers or enantiomers is less than 100%, and the content of the isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise specified, the term "isomer excess" or "enantiomeric excess" refers to the difference between the relative percentages of two isomers or two enantiomers. For example, if the content of one isomer or enantiomer is 90%, and the content of the other isomer or enantiomer is 10%, the isomer or enantiomer excess (ee value) is 80%.

Optically active (*R*)- and (*S*)-isomers, or *D* and *L* isomers can be prepared using chiral synthesis, chiral reagents, or other conventional techniques. If one kind of enantiomer of certain compound of the present disclosure is to be obtained, it can be prepared by asymmetric synthesis or derivative action of chiral auxiliary, wherein the enantiomer in resulting diastereomeric mixture is separated and the auxiliary group is cleaved to provide the pure desired enantiomer. Alternatively, when the molecule contains a basic functional group (such as amino) or an acidic functional group (such as carboxyl), a salt of a diastereoisomer is formed with an appropriate optically active acid or base, and then diastereomeric resolution is performed by conventional methods known in the art, and then the pure enantiomer is recovered. In addition, the enantiomer and the diastereoisomer are generally separated through chromatography which uses a chiral stationary phase and optionally combines with a chemical derivative method (such as carbamate generated from amine).

The compound of the present disclosure may contain an unnatural proportion of atomic isotope at one or more atoms that constitute the compound. For example, the compound can be radiolabeled with a radioactive isotope, such as tritium (³H), iodine-125 (¹²⁵I), or C-14 (¹⁴C). For another example, deuterated drugs can be formed by replacing hydrogen with deuterium, the bond formed by deuterium and carbon is stronger than that of ordinary hydrogen and carbon. Compared with non-deuterated drugs, deuterated drugs have the advantages of reduced toxic and side effects, increased drug stability, enhanced efficacy, extended biological half-life of drugs, etc. All isotopic variations of the compound of the present disclosure, whether radioactive or not, are encompassed within the scope of the present disclosure.

When the enumerative linking group does not indicate the direction for linking, the direction for linking is arbitrary, for example, the linking group L contained in 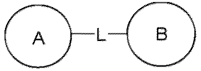 is -M-W-, then -M-W- can link ring A and ring B to form 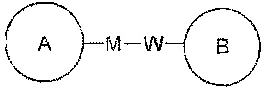 in the direction same as left-to-right reading order, or link ring A and ring B to form 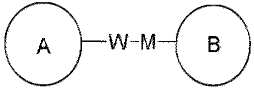 in the direction contrary to left-to-right reading order. A combination of the linking groups, substituents and/or variables thereof is allowed only when such combination can result in a stable compound.

Unless otherwise specified, when a group has one or more linkable sites, any one or more sites of the group can be linked to other groups through chemical bonds. When the linking site of the chemical bond is not positioned, and there is H atom at the linkable site, then the number of H atoms at the site will decrease correspondingly with the number of the chemical bonds linking thereto so as to meet the corresponding valence. The chemical bond between the site and other groups can be represented by a straight solid bond ( 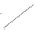 ), a straight dashed bond ( 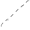 ), or a wavy line ( 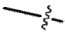 ). For example, the straight solid bond in -OCH₃ means that it is linked to other groups through the oxygen atom in the group; the straight dashed bond in 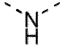 means that it is linked to other groups through the two ends of nitrogen atom in the group; the wave line in 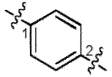 means that the phenyl group is linked to other groups through carbon atoms at position 1 and position 2.

Unless otherwise specified, the term "C₁₋₃ alkyl" refers to a linear or branched saturated hydrocarbon group consisting of 1 to 3 carbon atoms. The C₁₋₃ alkyl includes C₁₋₂ and C₂₋₃ alkyl, etc.; it can be monovalent (such as methyl), divalent (such as methylene), or multivalent (such as methine). Examples of C₁₋₃ alkyl include, but are not limited to, methyl (Me), ethyl (Et), propyl (including *n*-propyl and isopropyl), etc.

The structure of the polypeptides of the present disclosure can be confirmed by conventional methods known to those skilled in the art, and if the present disclosure involves an absolute configuration of a compound, then the absolute configuration can be confirmed by means of conventional techniques in the art. For example, in the case of single crystal X-ray diffraction (SXRD), diffraction intensity data are collected from the cultured single crystal using a Bruker D8 venture diffractometer with CuKα radiation as the light source and scanning mode: ϕ/ω scan, and after collecting the relevant data, the crystal structure is further analyzed by direct method (Shelxs97), so that the absolute configuration can be confirmed.

The polypeptide of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred embodiments include but are not limited to the examples of the present disclosure.

The solvent used in the present disclosure is commercially available.

The following abbreviations are used the present disclosure: aq represents aqueous, eq represents equivalent, equal amount; DCM represents dichloromethane; PE represents petroleum ether; DMSO represents dimethyl sulfoxide; MeOH represents methanol; Boc represents *tert*-butoxycarbonyl, which is an amine-protecting group; Dde represents (4,4-dimethyl-2,6-dioxocyclohexylidene)ethyl, which is a side chain protecting group for amino acids; r.t. represents room temperature; O/N represents overnight; THF represents tetrahydrofuran; Boc₂O represents di-*tert*-butyl dicarbonate; TFA represents trifluoroacetic acid; DIEA represents diisopropylethylamine; DMF represents *N*,*N*-dimethylformamide; HBTU represents benzotriazole-*N*,*N*,*N',N'*-tetramethyluronium hexafluorophosphate; HOBT represents 1-hydroxybenzotriazole; HOAT represents 1-hydroxy-7-azabenzotriazole; DIC represents *N,N'*-diisopropylcarbodiimide; DBU represents 1,8-diazabicyclo[5.4.0]undec-7-ene; PhSiH₃ represents phenylsilane; Pd(PPh₃)₄ represents tetrakis(triphenylphosphine)palladium; AEEA represents 2-(2-(2-aminoethoxy)ethoxy)acetic acid; DIEA represents diisopropylethylamine.

The compounds of the present disclosure are named according to the conventional naming principles in the art or by ChemDraw^{®} software, and the commercially available compounds use the supplier catalog names.

### DETAILED DESCRIPTION OF THE PREFERRED EMBODIMENT

The present disclosure is described in detail by the examples below, but it does not mean that there are any adverse restrictions on the present disclosure. The present disclosure has been described in detail herein, and its specific examples have also been disclosed; for those skilled in the art, it is obvious to make various modifications and improvements to the examples of the present disclosure without departing from the spirit and scope of the present disclosure.

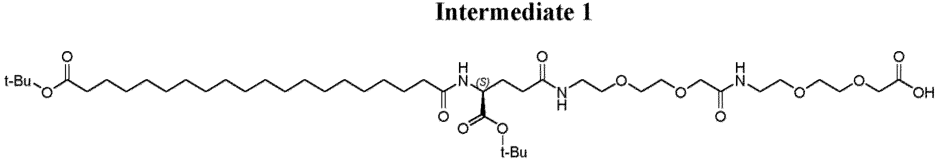

### 1. Resin loading

1.1 20.0 g of chloro(*o*-chlorophenyl)diphenylmethane (2-CTC Resin (degree of substitution S = 1.00 mmol/g)) and 7.72 g of Fmoc-AEEA-OH were weighed and added to a reaction column. Then DCM (40 mL) was added thereto, followed by the addition of 14.0 mL of DIEA to the reaction column and the reaction column was agitated with nitrogen for 2 h. Subsequently, 20.0 mL of MeOH was added to the reaction column and the reaction column was further agitated with nitrogen for 30 minutes. The waste was drained until no liquid flowed out. The reaction column was washed with DMF (300 mL) for 5 times, 1 min each time, and the waste was drained until no liquid flowed out.

1.2 20% piperidine/DMF (300 mL) was added to the reaction column, and the reaction column was agitated with nitrogen for 20 minutes. The waste was drained until no liquid flowed out. The reaction column was washed with DMF (100 mL) for 5 times, 1 min each time, and the waste was drained until no liquid flowed out. Ninhydrin was used for detection, with the resin appearing blue.

### 2. Coupling of amino acids

### 2.1 Coupling of Fmoc-AEEA-OH

1. Fmoc-AEEA-OH (3.0 eq) was weighed and added to the above resin, then DIEA (6.00 eq) was added, followed by the supplementary addition of 120 mL of DMF to the reaction column. The reaction column was agitated with nitrogen and then added with HBTU (2.85 eq) after the amino acids were dissolved. Nitrogen was adjusted to make the resin bulge evenly.
2. The reaction was carried out at 25°C for 20 min. Ninhydrin was used for detection, with the resin appearing colorless and transparent.
3. The reaction solution was drawn off. Then the reaction column was washed with DMF for 5 times, 1 min each time, and the waste was drained until no liquid flowed out.

### 2.2 Coupling of Fmoc-Glu-OtBu

1. 20% piperidine/DMF (300 mL) was added to the reaction column, and the reaction column was agitated with nitrogen for 20 minutes. The waste was drained until no liquid flowed out. Ninhydrin was used for detection, with the resin appearing blue.
2. Fmoc-Glu-OtBu (3.0 eq) was weighed and added to the above resin, then DIEA (3.00 eq) was added, followed by the supplementary addition of 300 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF (300 mL) for 5 times, 1 min each time, and the waste was drained until no liquid flowed out.

### 2.3 Coupling of 20-(tert-butoxy)-20-oxoicosanoic acid

1. 20% piperidine/DMF (300 mL) was added to the reaction column, and the reaction column was agitated with nitrogen for 20 minutes. The waste was drained until no liquid flowed out. The reaction column was washed with DMF (300 mL) for 5 times, 1 min each time, and the waste was drained until no liquid flowed out. Ninhydrin was used for detection, with the resin appearing blue.
2. 20-(*tert*-Butoxy)-20-oxoicosanoic acid (3.0 eq) was weighed and added to the above resin, then DIEA (6.00 eq) was added, followed by the supplementary addition of 300 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times, 1 min each time, and the waste was drained until no liquid flowed out.

### 3. Cleavage and crude peptide drying

### 3.1. A cleavage buffer was prepared according to the following volume:

Hexafluoroisopropanol (HFIP)/DCM = 20/80.

3.2. The prepared 400 mL of cleavage buffer was poured into a reactor containing dried peptide resin, agitated with gas for 20 minutes in the reactor, and filtered, and the filtrate was added to a flask. This operation was repeated twice. The collected cleavage buffer in the two operations was subjected to rotary evaporation until dryness to obtain a crude peptide **intermediate 1.**

Various types of polypeptides involved in the examples of the present disclosure are synthesized below according to standard solid-phase synthesis methods and purified by reversed-phase HPLC.

### Example 1

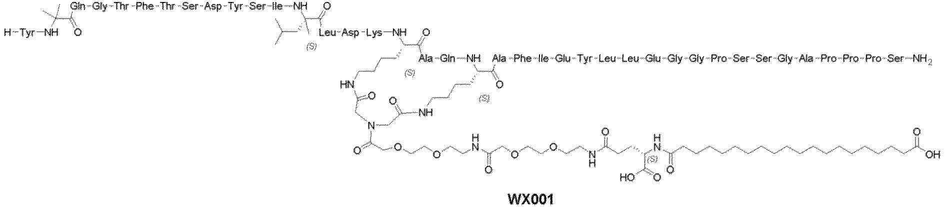

**1.** 1.78 g of 4-(2',4'-dimethoxyphenyl-Fmoc-aminomethyl)-phenoxyacetamido-norleucyl-MBHAresin (Rink Amide MBHAResin (degree of substitution Sub = 0.28 mmol/g)) was weighed and added to a reaction column. Then, DMF (50 mL) was added to the reaction column and the reaction column was agitated with nitrogen for 2 h. The waste was drained until no liquid flowed out. The reaction column was washed with DMF (50 mL) for 5 times, 1 min each time, and the waste was drained until no liquid flowed out.
**2.** 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.

### 3. Coupling of amino acids

### 3.1 Coupling of Fmoc-Ser(tBu)-OH

1. Fmoc-Ser(tBu)-OH (3.0 eq) was weighed and added to the above resin, then DIEA (6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
2. The reaction was carried out at 25°C for 0.5 h. Ninhydrin was used for detection, with the resin appearing colorless and transparent.
3. The reaction solution was drawn off. Then the reaction column was washed with DMF for 5 times (50 mL each time), 1 min each time, and the waste was drained until no liquid flowed out.

### 3.2 Coupling of Fmoc-Pro-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Pro-OH (3.0 eq) was weighed and added to the above resin, then DIEA (6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.3 Coupling of Fmoc-Pro-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Tetrachlorobenzoquinone was used for detection, with the resin appearing green.
2. Fmoc-Pro-OH (3.0 eq) was weighed and added to the above resin, DIEA (6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Tetrachlorobenzoquinone was used for detection, with the resin appearing colorless and transparent.
4. The reaction solution was drawn off. Then the reaction column was washed with DMF for 5 times, 1 min each time, and the waste was drained until no liquid flowed out.

### 3.4 Coupling of Fmoc-Pro-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Tetrachlorobenzoquinone was used for detection, with the resin appearing green.
2. Fmoc-Pro-OH (3.0 eq) was weighed and added to the above resin, then DIEA (6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Tetrachlorobenzoquinone was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.5 Coupling of Fmoc-Ala-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Tetrachlorobenzoquinone was used for detection, with the resin appearing green.
2. Fmoc-Ala-OH (3.0 eq) was weighed and added to the above resin, then DIEA (6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Tetrachlorobenzoquinone was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.6 Coupling of Fmoc-Gly-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Gly-OH (3.0 eq) was weighed and added to the above resin, then DIEA(6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.7 Coupling of Fmoc-Ser(tBu)-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Ser(tBu)-OH (3.0 eq) was weighed and added to the above resin, then DIEA (6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.8 Coupling of Fmoc-Ser(tBu)-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Ser(tBu)-OH (3.0 eq) was weighed and added to the above resin, then DIEA (6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.9 Coupling of Fmoc-Pro-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Pro-OH (3.0 eq) was weighed and added to the above resin, then DIEA (6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.10 Coupling of Fmoc-Gly-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Tetrachlorobenzoquinone was used for detection, with the resin appearing green.
2. Fmoc-Gly-OH (3.0 eq) was weighed and added to the above resin, then DIEA(6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Tetrachlorobenzoquinone was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.11 Coupling of Fmoc-Gly-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Gly-OH (3.0 eq) was weighed and added to the above resin, then DIEA(6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.12 Coupling of Fmoc-Glu(OtBu)-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Glu(OtBu)-OH (3.0 eq) was weighed and added to the above resin, then DIEA (6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.13 Coupling of Fmoc-Leu-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Leu-OH (3.0 eq) was weighed and added to the above resin, then DIEA (6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.14 Coupling of Fmoc-Leu-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Leu-OH (3.0 eq) was weighed and added to the above resin, DIEA (6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.15 Coupling of Fmoc-Tyr(tBu)-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Tyr(tBu)-OH (3.0 eq) was weighed and added to the above resin, then DIEA (6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.16 Coupling of Fmoc-Glu(OtBu)-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Glu(OtBu)-OH (3.0 eq) was weighed and added to the above resin, then DIEA (6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.17 Coupling of Fmoc-Ile-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Ile-OH (3.0 eq) was weighed and added to the above resin, then DIEA (6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.18 Coupling of Fmoc-Phe-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Phe-OH (3.0 eq) was weighed and added to the above resin, then DIEA(6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.19 Coupling of Fmoc-Ala-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Ala-OH (3.0 eq) was weighed and added to the above resin, then DIEA(6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.20 Coupling of Fmoc-Lys(Dde)-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Lys(Dde)-OH (3.0 eq) was weighed and added to the above resin, then DIEA (6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.21 Coupling of Fmoc-Gln(Trt)-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Gln(Trt)-OH (3.0 eq) was weighed and added to the above resin, then DIEA (6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.22 Coupling of Fmoc-Ala-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Ala-OH (3.0 eq) was weighed and added to the above resin, then DIEA (6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.23 Coupling of Fmoc-Lys(Alloc)-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Lys(Alloc)-OH (6.0 eq) was weighed and added to the above resin, then DIEA (12.0 eq) was added, followed by the supplementary addition of 10 mL of DMF to the reaction column. The reaction column was agitated with nitrogen and then added with HATU (5.70 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.24 Coupling of Fmoc-Lys(Boc)-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Lys(Boc)-OH (6.0 eq) was weighed and added to the above resin, then DIEA (12.0 eq) was added, followed by the supplementary addition of 10 mL of DMF to the reaction column. The reaction column was agitated with nitrogen and then added with HATU (5.70 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.25 Coupling of Fmoc-Asp(OtBu)-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Asp(OtBu)-OH (6.0 eq) was weighed and added to the above resin, then DIEA (12.0 eq) was added, followed by the supplementary addition of 10 mL of DMF to the reaction column. The reaction column was agitated with nitrogen and then added with HATU (5.70 eq) after the amino acids were dissolved. Nitrogen was adjusted to make the resin bulge evenly.
3. The reaction was carried out at 25°C for 2 h. Ninhydrin was used for detection, with the resin appearing blue.
4. The reaction solution was drawn off. Then the reaction column was washed with DMF for 5 times (50 mL each time), 1 min each time, and the waste was drained until no liquid flowed out.

### 3.26 Coupling of Fmoc-Leu-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Leu-OH (6.0 eq) was weighed and added to the above resin, HOAT (6.00 eq) was added, followed by the supplementary addition of 10 mL of DMF to the reaction column. The reaction column was agitated with nitrogen and then added with DIC (6.00 eq) after the amino acids and HOAT were dissolved. Nitrogen was adjusted to make the resin bulge evenly.
3. The reaction was carried out at 25°C for 1 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.27 Coupling of Fmoc-α-Me-Leu-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-α-Me-Leu-OH (6.0 eq) was weighed and added to the above resin, then DIEA (12.0 eq) was added, followed by the supplementary addition of 10 mL of DMF to the reaction column. The reaction column was agitated with nitrogen and then added with HATU (5.70 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.28 Coupling of Fmoc-Ile-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Ile-OH (6.0 eq) was weighed and added to the above resin, then DIEA (12.0 eq) was added, followed by the supplementary addition of 10 mL of DMF to the reaction column. The reaction column was agitated with nitrogen and then added with HATU (5.70 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.29 Coupling of Fmoc-Ser(tBu)-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Ser(tBu)-OH (6.0 eq) was weighed and added to the above resin, then DIEA (12.0 eq) was added, followed by the supplementary addition of 10 mL of DMF to the reaction column. The reaction column was agitated with nitrogen and then added with HATU (5.70 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.30 Coupling of Fmoc-Tyr(tBu)-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Tyr(tBu)-OH (6.0 eq) was weighed and added to the above resin, then DIEA (12.0 eq) was added, followed by the supplementary addition of 10 mL of DMF to the reaction column. The reaction column was agitated with nitrogen and then added with HATU (5.70 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.31 Coupling of Fmoc-Asp(OtBu)-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Asp(OtBu)-OH (3.0 eq) was weighed and added to the above resin, then DIEA (6.00 eq) was added, followed by the supplementary addition of 10 mL of DMF to the reaction column. The reaction column was agitated with nitrogen and then added with HATU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.32 Coupling of Fmoc-Ser(tBu)-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Ser(tBu)-OH (6.0 eq) was weighed and added to the above resin, then HOBT (6.00 eq) was added, followed by the supplementary addition of 10 mL of DMF to the reaction column. The reaction column was agitated with nitrogen and then added with DIC (6.00 eq) after the amino acids and HOBT were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.33 Coupling of Fmoc-Thr(tBu)-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Thr(tBu)-OH (3.0 eq) was weighed and added to the above resin, then DIEA (6.00 eq) was added, followed by the supplementary addition of 10 mL of DMF to the reaction column. The reaction column was agitated with nitrogen and then added with HATU (2.85 eq) after the amino acids were dissolved. Nitrogen was adjusted to make the resin bulge evenly.
3. The reaction was carried out at 25°C overnight. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.34 Coupling of Fmoc-Phe-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Phe-OH (6.0 eq) was weighed and added to the above resin, then HOBT (6.00 eq) was added, followed by the supplementary addition of 10 mL of DMF to the reaction column. The reaction column was agitated with nitrogen and then added with DIC (6.00 eq) after the amino acids and HOBT were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.35 Coupling of Fmoc-Thr(tBu)-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Thr(tBu)-OH (3.0 eq) was weighed and added to the above resin, then DIEA (6.00 eq) was added, followed by the supplementary addition of 10 mL of DMF to the reaction column. The reaction column was agitated with nitrogen and then added with HATU (2.85 eq) after the amino acids were dissolved. Nitrogen was adjusted to make the resin bulge evenly.
3. The reaction was carried out at 25°C overnight. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.36 Coupling of Fmoc-Gly-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Boc-His(Trt)-OH (6.0 eq) was weighed and added to the above resin, then DIEA (12.0 eq) was added, followed by the supplementary addition of 10 mL of DMF to the reaction column. The reaction column was agitated with nitrogen and then added with HATU (5.70 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.37 Coupling of Fmoc-Gln(Trt)-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Gln(Trt)-OH (6.0 eq) was weighed and added to the above resin, then DIEA (12.0 eq) was added, followed by the supplementary addition of 10 mL of DMF to the reaction column. The reaction column was agitated with nitrogen and then added with HATU (5.70 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.38 Coupling of Fmoc-Aib-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Aib-OH (6.0 eq) was weighed and added to the above resin, then DIEA(12.0 eq) was added, followed by the supplementary addition of 10 mL of DMF to the reaction column. The reaction column was agitated with nitrogen and then added with HATU (5.70 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.39 Coupling of Boc-Tyr(tBu)-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Tetrachlorobenzoquinone was used for detection, with the resin appearing green.
2. Boc-Tyr(tBu)-OH (6.0 eq) was weighed and added to the above resin, then DIEA (12.0 eq) was added, followed by the supplementary addition of 10 mL of DMF to the reaction column. The reaction column was agitated with nitrogen and then added with HATU (5.70 eq) after the amino acids were dissolved. Nitrogen was adjusted to make the resin bulge evenly.
3. The reaction was carried out at 25°C for 0.5 h. Tetrachlorobenzoquinone was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.40 Removal of Alloc

1. PhSiH₃ (10.0 eq) and DCM (10mL) were added to the reaction column. The reaction column was agitated with nitrogen, then added with Pd(PPh₃)₄ (0.1 eq), and then agitated with nitrogen for 20 minutes. The reaction was carried out twice, and the waste was drained until no liquid flowed out.
2. The reaction column was washed with DMF for 5 times (50 mL each time), 1 min each time, and the waste was drained until no liquid flowed out.

### 3.41 Coupling of Fmoc-Ida-OH

1. Fmoc-Ida-OH (CAS#: 112918-82-8) (6.0 eq) was weighed and added to the above resin, then DIEA (12.0 eq) was added, followed by the supplementary addition of 10 mL of DMF to the reaction column. The reaction column was agitated with nitrogen and then added with HBTU (5.70 eq) after the amino acids were dissolved. Nitrogen was adjusted to make the resin bulge evenly.
2. The reaction was carried out at 25°C for 0.5 h. Ninhydrin was used for detection, with the resin appearing colorless and transparent.
3. The reaction solution was drawn off. Then the reaction column was washed with DMF for 5 times (50 mL each time), 1 min each time, and the waste was drained until no liquid flowed out.

### 3.42 Coupling of intermediate 1

1. 10% DBU/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. **Intermediate 1** (1.50 eq) was weighed and added to the above resin, then DIEA (3.00 eq) was added, followed by the supplementary addition of 10 mL of DMF to the reaction column. The reaction column was agitated with nitrogen and then added with HBTU (1.45 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.43 Removal of Dde

1. 3% Hydrazine hydrate/DMF (50 mL) was added to the reaction column, and the reaction column was agitated with nitrogen for 15 minutes. The waste was drained. The reaction column was washed with DMF (50 mL) for 5 times, 1 min each time, and the waste was drained until no liquid flowed out. Ninhydrin was used for detection, with the resin appearing blue.

### 3.44 Closing of amide ring

1. DIEA (3.0 eq) was added to a solution of the above resin in DMF, then HATU (1.5 eq) which was dissolved in DMF was slowly added dropwise to the reaction column, and then the reaction column was agitated with nitrogen. Nitrogen was adjusted to make the resin bulge evenly.
2. The reaction was carried out at 25°C for 0.5 h. Ninhydrin was used for detection, with the resin appearing colorless and transparent.
3. The reaction solution was drawn off. Then the reaction column was washed with DMF for 5 times (50 mL each time), 1 min each time, and the waste was drained until no liquid flowed out.
4. The resin was shrunk with MeOH (50 mL), 3 min each time, and the waste was drained until no liquid flowed out. Then the resin was poured out and dried for further use.

### 4. Cleavage and crude peptide drying

### 4.1. A cleavage buffer was prepared according to the following volume:

TFA/ Tis/ H₂O/ Mpr = 90/2.5/2.5/5.

4.2 The dried peptide resin was added to the prepared cleavage buffer. The mixture was shaken on a shaker for 2.5 h and then filtered, and the filtrate was added to 10 times the volume of ice-cold isopropyl ether. The resulting mixture was centrifuged, and then washed with isopropyl ether 5 times. After drying in vacuum for 2 h, crude peptide was obtained and purified to obtain WX001. The molecular weight of the polypeptide was confirmed by ESI-MS, with a calculated value of [M+3H]/3 as 1673.2 and a detected value of 1673.0.

### Example 2

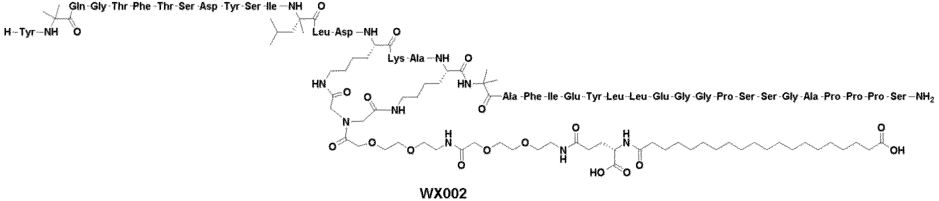

With reference to the synthesis of **WX001,** peptide **WX002** was obtained. The molecular weight of the polypeptide was confirmed by ESI-MS, with a calculated value of [M+3H]/3 as 1658.9 and a detected value of 1658.7.

### Example 3

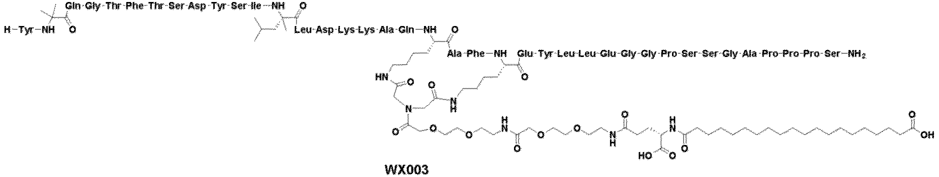

With reference to the synthesis of **WX001,** peptide **WX003** was obtained. The molecular weight of the polypeptide was confirmed by ESI-MS, with a calculated value of [M+3H]/3 as 1678.2 and a detected value of 1678.1.

### Example 4

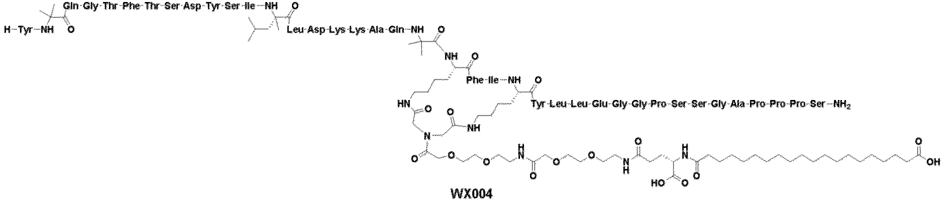

With reference to the synthesis of **WX001,** peptide **WX004** was obtained. The molecular weight of the polypeptide was confirmed by ESI-MS, with a calculated value of [M+3H]/3 as 1677.6 and a detected value of 1677.6.

### Example 5

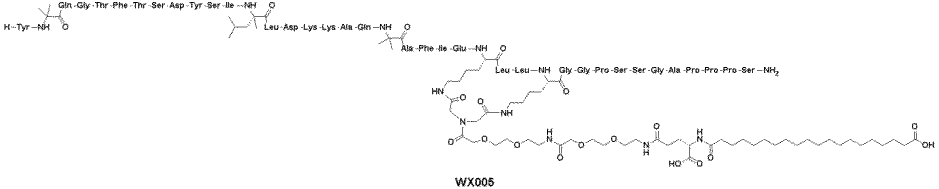

**1.** 2 g of 4-(2',4'-dimethoxyphenyl-Fmoc-aminomethyl)-phenoxyacetamido-norleucyl-MBHAresin (Rink Amide MBHAResin (degree of substitution Sub = 0.28 mmol/g)) was weighed and added to a reaction column. Then, DMF (50 mL) was added to the reaction column and the reaction column was agitated with nitrogen for 2 h. The waste was drained until no liquid flowed out. The reaction column was washed with DMF (50 mL) for 5 times, 1 min each time, and the waste was drained until no liquid flowed out.
**2.** 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.

### 3. Coupling of amino acids

### 3.1 Coupling of Fmoc-Ser(tBu)-OH

1. Fmoc-Ser(tBu)-OH (3.0 eq) was weighed and added to the above resin, then DIEA (6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
2. The reaction was carried out at 25°C for 0.5 h. Ninhydrin was used for detection, with the resin appearing colorless and transparent.
3. The reaction solution was drawn off. Then the reaction column was washed with DMF for 5 times (50 mL each time), 1 min each time, and the waste was drained until no liquid flowed out.

### 3.2 Coupling of Fmoc-Pro-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Pro-OH (3.0 eq) was weighed and added to the above resin, then DIEA (6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.3 Coupling of Fmoc-Pro-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Tetrachlorobenzoquinone was used for detection, with the resin appearing green.
2. Fmoc-Pro-OH (3.0 eq) was weighed and added to the above resin, then DIEA (6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Tetrachlorobenzoquinone was used for detection, with the resin appearing colorless and transparent.
4. The reaction solution was drawn off. Then the reaction column was washed with DMF for 5 times, 1 min each time, and the waste was drained until no liquid flowed out.

### 3.4 Coupling of Fmoc-Pro-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Tetrachlorobenzoquinone was used for detection, with the resin appearing green.
2. Fmoc-Pro-OH (3.0 eq) was weighed and added to the above resin, then DIEA (6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Tetrachlorobenzoquinone was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.5 Coupling of Fmoc-Ala-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Tetrachlorobenzoquinone was used for detection, with the resin appearing green.
2. Fmoc-Ala-OH (3.0 eq) was weighed and added to the above resin, then DIEA(6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Tetrachlorobenzoquinone was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.6 Coupling of Fmoc-Gly-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Gly-OH (3.0 eq) was weighed and added to the above resin, then DIEA(6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.7 Coupling of Fmoc-Ser(tBu)-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Ser(tBu)-OH (3.0 eq) was weighed and added to the above resin, then DIEA (6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.8 Coupling of Fmoc-Ser(tBu)-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Ser(tBu)-OH (3.0 eq) was weighed and added to the above resin, then DIEA (6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.9 Coupling of Fmoc-Pro-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Pro-OH (3.0 eq) was weighed and added to the above resin, then DIEA (6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.10 Coupling of Fmoc-Gly-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Tetrachlorobenzoquinone was used for detection, with the resin appearing green.
2. Fmoc-Gly-OH (3.0 eq) was weighed and added to the above resin, then DIEA (6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Tetrachlorobenzoquinone was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.11 Coupling of Fmoc-Gly-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Gly-OH (3.0 eq) was weighed and added to the above resin, then DIEA(6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.12 Coupling of Fmoc-Lys(Dde)-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Lys(Dde)-OH (3.0 eq) was weighed and added to the above resin, then DIEA (6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.13 Coupling of Fmoc-Leu-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Leu-OH (3.0 eq) was weighed and added to the above resin, then DIEA(6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.14 Coupling of Fmoc-Leu-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Leu-OH (3.0 eq) was weighed and added to the above resin, then DIEA(6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.15 Coupling of Fmoc-Lys(Alloc)-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Lys(Alloc)-OH (6.0 eq) was weighed and added to the above resin, then DIEA (12.0 eq) was added, followed by the supplementary addition of 10 mL of DMF to the reaction column. The reaction column was agitated with nitrogen and then added with HATU (5.70 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.16 Coupling of Fmoc-Glu(OtBu)-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Glu(OtBu)-OH (3.0 eq) was weighed and added to the above resin, then DIEA (6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.17 Coupling of Fmoc-Ile-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Ile-OH (3.0 eq) was weighed and added to the above resin, then DIEA (6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.18 Coupling of Fmoc-Phe-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Phe-OH (3.0 eq) was weighed and added to the above resin, then DIEA(6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.19 Coupling of Fmoc-Ala-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Ala-OH (3.0 eq) was weighed and added to the above resin, then DIEA(6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.20 Coupling of Fmoc-Aib-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Aib-OH (6.0 eq) was weighed and added to the above resin, then DIEA(12.0 eq) was added, followed by the supplementary addition of 10 mL of DMF to the reaction column. The reaction column was agitated with nitrogen and then added with HATU (5.70 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.21 Coupling of Fmoc-Gln(Trt)-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Gln(Trt)-OH (3.0 eq) was weighed and added to the above resin, then DIEA (6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.22 Coupling of Fmoc-Ala-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Ala-OH (3.0 eq) was weighed and added to the above resin, then DIEA (6.00 eq) was added, followed by the supplementary addition of 10 mL of DMF to the reaction column. The reaction column was agitated with nitrogen and then added with HBTU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.23 Coupling of Fmoc-Lys(Boc)-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Lys(Boc)-OH (6.0 eq) was weighed and added to the above resin, then DIEA (12.0 eq) was added, followed by the supplementary addition of 10 mL of DMF to the reaction column. The reaction column was agitated with nitrogen and then added with HATU (5.70 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.24 Coupling of Fmoc-Lys(Boc)-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Lys(Boc)-OH (6.0 eq) was weighed and added to the above resin, then DIEA (12.0 eq) was added, followed by the supplementary addition of 10 mL of DMF to the reaction column. The reaction column was agitated with nitrogen and then added with HATU (5.70 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.25 Coupling of Fmoc-Asp(OtBu)-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Asp(OtBu)-OH (6.0 eq) was weighed and added to the above resin, then DIEA (12.0 eq) was added, followed by the supplementary addition of 10 mL of DMF to the reaction column. The reaction column was agitated with nitrogen and then added with HATU (5.70 eq) after the amino acids were dissolved. Nitrogen was adjusted to make the resin bulge evenly.
3. The reaction was carried out at 25°C for 2 h. Ninhydrin was used for detection, with the resin appearing blue.
4. The reaction solution was drawn off. Then the reaction column was washed with DMF for 5 times (50 mL each time), 1 min each time, and the waste was drained until no liquid flowed out.

### 3.26 Coupling of Fmoc-Leu-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Leu-OH (6.0 eq) was weighed and added to the above resin, HOAT (6.00 eq) was added, followed by the supplementary addition of 10 mL of DMF to the reaction column. The reaction column was agitated with nitrogen and then added with DIC (6.00 eq) after the amino acids and HOAT were dissolved. Nitrogen was adjusted to make the resin bulge evenly.
3. The reaction was carried out at 25°C for 1 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.27 Coupling of Fmoc-α-Me-Leu-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-α-Me-Leu-OH (6.0 eq) was weighed and added to the above resin, then DIEA (12.0 eq) was added, followed by the supplementary addition of 10 mL of DMF to the reaction column. The reaction column was agitated with nitrogen and then added with HATU (5.70 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.28 Coupling of Fmoc-Ile-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Ile-OH (6.0 eq) was weighed and added to the above resin, then DIEA (12.0 eq) was added, followed by the supplementary addition of 10 mL of DMF to the reaction column. The reaction column was agitated with nitrogen and then added with HATU (5.70 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.29 Coupling of Fmoc-Ser(tBu)-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Ser(tBu)-OH (6.0 eq) was weighed and added to the above resin, then DIEA (12.0 eq) was added, followed by the supplementary addition of 10 mL of DMF to the reaction column. The reaction column was agitated with nitrogen and then added with HATU (5.70 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.30 Coupling of Fmoc-Tyr(tBu)-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Tyr(tBu)-OH (6.0 eq) was weighed and added to the above resin, then DIEA (12.0 eq) was added, followed by the supplementary addition of 10 mL of DMF to the reaction column. The reaction column was agitated with nitrogen and then added with HATU (5.70 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.31 Coupling of Fmoc-Asp(OtBu)-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Asp(OtBu)-OH (3.0 eq) was weighed and added to the above resin, then DIEA (6.00 eq) was added, followed by the supplementary addition of 10 mL of DMF to the reaction column. The reaction column was agitated with nitrogen and then added with HATU (2.85 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.32 Coupling of Fmoc-Ser(tBu)-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Ser(tBu)-OH (6.0 eq) was weighed and added to the above resin, then HOBT (6.00 eq) was added, followed by the supplementary addition of 10 mL of DMF to the reaction column. The reaction column was agitated with nitrogen and then added with DIC (6.00 eq) after the amino acids and HOBT were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.33 Coupling of Fmoc-Thr(tBu)-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Thr(tBu)-OH (3.0 eq) was weighed and added to the above resin, then DIEA (6.00 eq) was added, followed by the supplementary addition of 10 mL of DMF to the reaction column. The reaction column was agitated with nitrogen and then added with HATU (2.85 eq) after the amino acids were dissolved. Nitrogen was adjusted to make the resin bulge evenly.
3. The reaction was carried out at 25°C overnight. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.34 Coupling of Fmoc-Phe-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Phe-OH (6.0 eq) was weighed and added to the above resin, then HOBT (6.00 eq) was added, followed by the supplementary addition of 10 mL of DMF to the reaction column. The reaction column was agitated with nitrogen and then added with DIC (6.00 eq) after the amino acids and HOBT were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.35 Coupling of Fmoc-Thr(tBu)-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Thr(tBu)-OH (3.0 eq) was weighed and added to the above resin, then DIEA (6.00 eq) was added, followed by the supplementary addition of 10 mL of DMF to the reaction column. The reaction column was agitated with nitrogen and then added with HATU (2.85 eq) after the amino acids were dissolved. Nitrogen was adjusted to make the resin bulge evenly.
3. The reaction was carried out at 25°C overnight. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.36 Coupling of Fmoc-Gly-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Boc-His(Trt)-OH (6.0 eq) was weighed and added to the above resin, then DIEA (12.0 eq) was added, followed by the supplementary addition of 10 mL of DMF to the reaction column. The reaction column was agitated with nitrogen and then added with HATU (5.70 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.37 Coupling of Fmoc-Gln(Trt)-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Gln(Trt)-OH (6.0 eq) was weighed and added to the above resin, then DIEA (12.0 eq) was added, followed by the supplementary addition of 10 mL of DMF to the reaction column. The reaction column was agitated with nitrogen and then added with HATU (5.70 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.38 Coupling of Fmoc-Aib-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. Fmoc-Aib-OH (6.0 eq) was weighed and added to the above resin, then DIEA(12.0 eq) was added, followed by the supplementary addition of 10 mL of DMF to the reaction column. The reaction column was agitated with nitrogen and then added with HATU (5.70 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.39 Coupling of Boc-Tyr(tBu)-OH

1. 20% piperidine/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Tetrachlorobenzoquinone was used for detection, with the resin appearing green.
2. Boc-Tyr(tBu)-OH (6.0 eq) was weighed and added to the above resin, then DIEA (12.0 eq) was added, followed by the supplementary addition of 10 mL of DMF to the reaction column. The reaction column was agitated with nitrogen and then added with HATU (5.70 eq) after the amino acids were dissolved. Nitrogen was adjusted to make the resin bulge evenly.
3. The reaction was carried out at 25°C for 0.5 h. Tetrachlorobenzoquinone was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.40 Removal of Alloc

1. PhSiH₃ (10.0 eq) and DCM (10mL) were added to the reaction column. The reaction column was agitated with nitrogen, then added with Pd(PPh₃)₄ (0.1 eq), and then agitated with nitrogen for 20 minutes. The reaction was carried out twice, and the waste was drained until no liquid flowed out.
2. The reaction column was washed with DMF for 5 times (50 mL each time), 1 min each time, and the waste was drained until no liquid flowed out.

### 3.41 Coupling of Fmoc-Ida-OH

1. Fmoc-Ida-OH (6.0 eq) was weighed and added to the above resin, then DIEA (12.0 eq) was added, followed by the supplementary addition of 10 mL of DMF to the reaction column. The reaction column was agitated with nitrogen and then added with HBTU (5.70 eq) after the amino acids were dissolved. Nitrogen was adjusted to make the resin bulge evenly.
2. The reaction was carried out at 25°C for 0.5 h. Ninhydrin was used for detection, with the resin appearing colorless and transparent.
3. The reaction solution was drawn off. Then the reaction column was washed with DMF for 5 times (50 mL each time), 1 min each time, and the waste was drained until no liquid flowed out.

### 3.42 Coupling of intermediate 1

1. 10% DBU/DMF (50 mL) was added to the reaction column, and the reaction column was agitated with nitrogen for 20 minutes. The waste was drained until no liquid flowed out. The reaction column was washed with DMF (50 mL) for 5 times, 1 min each time, and the waste was drained until no liquid flowed out. Ninhydrin was used for detection, with the resin appearing blue.
2. **Intermediate 1** (1.50 eq) was weighed and added to the above resin, then DIEA (3.00 eq) was added, followed by the supplementary addition of 10 mL of DMF to the reaction column. The reaction column was agitated with nitrogen and then added with HBTU (1.45 eq) after the amino acids were dissolved. Nitrogen was adjusted to make the resin bulge evenly.
3. The reaction was carried out at 25°C for 0.5 h. Ninhydrin was used for detection, with the resin appearing colorless and transparent.
4. The reaction solution was drawn off. Then the reaction column was washed with DMF for 5 times (50 mL each time), 1 min each time, and the waste was drained until no liquid flowed out.

### 3.43 Removal of Dde

1. 3% Hydrazine hydrate/DMF (50 mL) was added to the reaction column, and the reaction column was agitated with nitrogen for 15 minutes. The waste was drained. The reaction column was washed with DMF (50 mL) for 5 times, 1 min each time, and the waste was drained until no liquid flowed out. Ninhydrin was used for detection, with the resin appearing blue.

### 3.44 Closing of amide ring

1. DIEA (3.0 eq) was added to a solution of the above resin in DMF, then HATU (1.5 eq) which was dissolved in DMF was slowly added dropwise to the reaction column, and then the reaction column was agitated with nitrogen. Nitrogen was adjusted to make the resin bulge evenly.
2. The reaction was carried out at 25°C for 0.5 h. Ninhydrin was used for detection, with the resin appearing colorless and transparent.
3. The reaction solution was drawn off. Then the reaction column was washed with DMF for 5 times (50 mL each time), 1 min each time, and the waste was drained until no liquid flowed out.
4. The resin was shrunk with MeOH (50 mL), 3 min each time, and the waste was drained until no liquid flowed out. Then the resin was poured out and dried for further use.

### 4. Cutting and crude peptide drying

### 4.1. A cleavage buffer was prepared according to the following volume:

TFA/ Tis/ H₂O/ Mpr = 90/2.5/2.5/5.

The dried peptide resin was added to the prepared cleavage buffer. The mixture was shaken on a shaker for 2.5 hours and then filtered, and the filtrate was added to 10 times the volume of ice-cold isopropyl ether. The resulting mixture was centrifuged, and then washed with isopropyl ether 5 times. After drying in vacuum for 2 h, crude peptide was obtained and purified to obtain polypeptide WX005. The molecular weight of the polypeptide was confirmed by ESI-MS, with a calculated value of [M+3H]/3 as 1646.9 and a detected value of 1646.9.

### Example 6

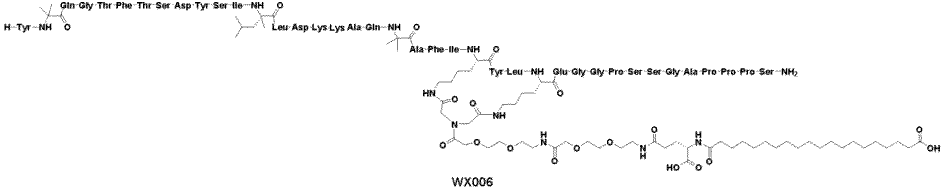

With reference to the synthesis of **WX001**, peptide **WX006** was obtained. The molecular weight of the polypeptide was confirmed by ESI-MS, with a calculated value of [M+3H]/3 as 1663.5 and a detected value of 1663.3.

### Biological test data

### Experimental example 1: In vitro GLP-1R/GIPR/GCGR agonistic activity test

### A: Main materials:

### 1) Cell line

The cell lines were constructed by WuXi AppTec (Shanghai) Co., Ltd. Details are shown in table 1.

**Table 1**

| Target | Host cell | Clone |
|---|---|---|
| GLP-1R | HEK293 | N/A |
| GCGR | HEK293 | N/A |
| GIPR | CHO | N/A |

### 2) Reagents and consumables are shown in Table 2:

**Table 2**

| Name | Batch | Cat. No. | Manufacturer |
|---|---|---|---|
| cAMP assay kit | 29F | 62AM4PEJ | Cisbio |
| 1M HEPES | 2120919 | 15630-106 | Invitrogen |
| Hanks' Balanced Salt Solution (HBSS) | 2185775 | 14025 | Invitrogen |
| Human Serum Albumin (HSA) | SLCF7301 | A1653-10G | Sigma |
| Casein | SLCC9458 | C4765-10 mL | Sigma |
| 3-Isobutyl-1-methylxanthine (IBMX) | STBF6061V | I5879-5G | Sigma |
| ECHO qualified 384-well plate | 0006433672 | PP-0200 | Labcyte |
| OptiPlate-384 | 8210-19481 | 6007299 | PerkinElmer |

### 3) Instruments are shown in Table 3:

**Table 3**

| Name | Model | Manufacturer |
|---|---|---|
| EnVision | envision2014 | PerkinElmer |
| Vi-cell counter | Vi-CELL^{™} XR Cell Viability Analyzer | Beckman |
| Bravo | Bravo V11 | Agilent |
| ECHO | ECHO 555 | Labcyte |
| Centrifuge | Allegra^{™} 25R Centrifuge | Beckman |

### B. Method

### I) Experimental materials

### The experimental buffers are shown in Table 4:

**Table 4**

| **Reagent** | **Stock concentration** | **Volume** | **Final concentration** |
|---|---|---|---|
| Hanks' Balanced Salt Solution | 1x | 48.7 mL/ | ≈ 1x |
| | | 44.7mL | |
| HEPES buffer | 1 mol/L | 250 µL | 5 mmol/L |
| 5% Casein solution (HEPES)/ 10% human serum albumin solution (HSA) | 5%/ 10% | 1000 µL/ 5000 µL | 0.10%/ 1% |
| 3 -Isobutyl-1 -methylxanthine (IBMX) | 500 mmol/L | 50 µL | 0.5 mmol/L |

### The preparation of assay reagents is shown in Table 5:

**Table 5**

| **Reagent** | **Storage concentration** | **Volume** | **Final concentration** |
|---|---|---|---|
| Cell lysate | 1x | 9.5 mL | ≈ 1x |
| D2-cAMP solution | 40x | 250 µL | 1x |
| cAMP-antibody solution | 40x | 250 µL | 1x |

### II) Experimental methods

a) Preparation of compound plates:
   The compounds to be tested were diluted 4-fold at 10 wells, starting from an initial concentration of 30 µM. The dilution was completed by Bravo.
b) Transfer of compounds:
   1) 100 nL of compounds were transferred to an OptiPlate-384 plate by Echo.
   2) The OptiPlate-384 plate was centrifuged at 1000 rpm for 5 seconds.
c) Preparation of cell suspension
   1) A GLP-1R/GIPR/GCGR cell cryopreservation tube was placed in warm water at 37°C for rapid thawing.
   2) The cell suspension was transferred into a 15 mL Transfer centrifugal tube and rinsed with 10 mL of HBSS gently.
   3) The centrifugal tube was centrifuged at 1000 rpm at room temperature for 1 min.
   4) The supernatant was discarded.
   5) The bottom cells were gently dispersed, then gently rinsed with 10 mL of HBSS, the centrifugal tube was centrifuged to sediment the cells, and finally the cells were resuspended in an experimental buffer.
   6) Cell density and viability were measured by Vi-cell.
   7) The GLP-1R/GIPR/GCGR cells were diluted with the experimental buffer to a concentration of 2.0 * 10⁵/mL.
   8) 100 nL of the diluted cell suspension was transferred into the OptiPlate-384 plate.
   9) The cells were incubated at room temperature for 30 min.
d) An assay reagent was added:
   1) 10 µL of 800 nM gradient diluted cAMP standard was added into the empty wells of the OptiPlate-384 plate.
   2) 10 µL of cAMP assay reagent was added.
   3) The OptiPlate-384 plate was sealed with TopSeal-A film and incubated at room temperature for 60 min.

The TopSeal-A film was removed, and readout was performed on EnVision.

### C. The experimental results are shown in Table 6:

**Table 6**

| **Polypeptide** | Activity test result | | |
|---|---|---|---|
| | GLP-1 EC₅₀ (nM) | GIP-1 EC₅₀ (nM) | GCG EC₅₀ (nM) |
| WX001 | 0.072 | 0.13 | 0.55 |
| WX002 | 0.095 | 0.57 | 1.0 |
| WX003 | 0.46 | 0.61 | > 20 |
| WX005 | 0.063 | 0.14 | 1.4 |

**Conclusion:** The polypeptides of the present disclosure exhibit strong agonistic activity on GLP-1R/GIPR. Among them, different peptides have great differences in their agonistic activity towards GCG, which will be instructive for the study of the balance of tri-target activity.

### Experimental example 2: Pharmacokinetic evaluation in rats

### A. Experimental purpose

To test the *in vivo* pharmacokinetics of the polypeptides of the present disclosure in SD rats.

### B. Experimental operations

The pharmacokinetic characteristics of the polypeptides of the present disclosure after subcutaneous injection in rodents were tested according to SOP. In the experiment, the polypeptide was prepared into a clear solution, which was administered to rats by single subcutaneous injection (SC, 0.048 mpk). The vehicle for injection was citrate buffer (20 mM, pH = 7). The whole blood was collected and prepared to obtain the plasma. The drug concentration was analyzed by LC-MS/MS method, and the pharmacokinetic parameters were calculated using Phoenix WinNonlin software.

### C. Experimental results

The experimental results are shown in Table 7:

**Table 7 Pharmacokinetic test results**

| **Polypeptide No.** | **Maximum plasma concentration (nM)** | **Tₘₐₓ (h)** | **Half-life T_{1/2} (h)** | **SC concentration integral AUC_{0-72 h} (nM.hr)** |
|---|---|---|---|---|
| **WX001** | 17 | 24 | 25 | 718 |
| **WX002** | 30 | 20 | 14 | 1061 |
| **WX005** | 26 | 28 | 23 | 1210 |

Conclusion: The polypeptides of the present disclosure exhibit excellent pharmacokinetic properties in rats.

### Experimental example 3: Pharmacokinetic evaluation in mice

### A. Experimental purpose

To test the *in vivo* pharmacokinetics of the polypeptides of the present disclosure in C57BL/6 mice.

### B. Experimental operations

The pharmacokinetic characteristics of the polypeptides of the present disclosure after intravenous injection and subcutaneous injection in rodents were tested according to SOP. In the experiment, the polypeptide was prepared into a clear solution, which was administered to mice by single subcutaneous injection (SC, 0.048 mpk). The vehicle for subcutaneous injection was citrate buffer (20 mM, pH = 7). The whole blood was collected and prepared to obtain the plasma. The drug concentration was analyzed by LC-MS/MS method, and the pharmacokinetic parameters were calculated using Phoenix WinNonlin software.

### C. Experimental results

The experimental results are shown in Table 8:

**Table 8 Pharmacokinetic test results**

| **Polypeptide No.** | **Maximum plasma concentration (nM)** | **Tₘₐₓ (h)** | **Half-life T_{1/2} (h)** | **SC concentration integral AUC₀₋₇₂ₕ (nM.hr)** |
|---|---|---|---|---|
| **WX001** | 58 | 12 | 16 | 1826 |
| **WX002** | 69 | 8 | 10 | 2006 |
| **WX005** | 72 | 12 | 15 | 2131 |

Conclusion: The polypeptides of the present disclosure exhibit excellent pharmacokinetic properties in mice.

### Experimental example 4: Pharmacokinetic evaluation in cynomolgus macaques

### A. Experimental purpose

To test the *in vivo* pharmacokinetics of the polypeptides of the present disclosure in cynomolgus macaques.

### B. Experimental operations

The pharmacokinetic characteristics of the polypeptides of the present disclosure after intravenous injection and subcutaneous injection in mammals were tested according to SOP. In the experiment, the polypeptide was prepared into a clear solution, which was administered to cynomolgus macaques by single subcutaneous injection (SC, 0.02 mpk). The vehicle for subcutaneous injection was citrate buffer (20 mM, pH = 7). The whole blood was collected and prepared to obtain the plasma. The drug concentration was analyzed by LC-MS/MS method, and the pharmacokinetic parameters were calculated using Phoenix WinNonlin software.

### C. Experimental results

The experimental results are shown in Table 9:

**Table 9 Pharmacokinetic test results**

| **Polypeptide No.** | **Maximum plasma concentration (nM)** | **Tₘₐₓ (h)** | **Half-life T_{1/2} (h)** | **SC concentration integral AUC₀₋₂₄₀ₕ (nM.hr)** |
|---|---|---|---|---|
| **WX001** | 49 | 32 | 61 | 5294 |
| **WX002** | 54 | 24 | 52 | 5100 |
| **WX005** | 43 | 48 | 100 | 5814 |

Conclusion: The polypeptides of the present disclosure exhibit excellent pharmacokinetic properties in monkeys.

### Experimental example 5: Efficacy study in DIO mice-in vivo efficacy evaluation

### A. Experimental purpose

To study the weight loss effect of the polypeptides of the present disclosure in DIO mice.

### B. Experimental operations

1. After the DIO mice arrived at the WuXi AppTec facility, they were housed in an animal breeding room with strictly controlled environmental conditions. The temperature of the breeding room was maintained between 20 and 24°C, and the humidity was maintained between 30 and 70%. The temperature and humidity in the breeding room were monitored in real time with a thermohygrometer, and the temperature and humidity were recorded twice a day (once in the morning and once in the afternoon). The lighting in the animal breeding room was controlled by an electronic timed lighting system. The lights were turned on for 12 hours and turned off for 12 hours every day (on at 7:00 am and off at 19:00 pm). During the experiment, the animals were housed individually in cages, and toys were provided in each cage. The animals had free access to food (growth/breeding feed for rats and mice) and water during the experiment.
2. Each group of animals was subcutaneously injected with vehicle (20 mM sodium citrate buffer, pH = 7.0) and polypeptide of the present disclosure (10 nmol/kg). The administration was performed at 9:30 a.m., once every three days, for a period of 22 days.

### C. Experimental results

The experimental results are shown in Table 10:

**Table 10 The efficacy of the polypeptides of the present disclosure in DIO mice**

| **Polypeptide No.** | **WX001** | **WX002** | **WX005** |
|---|---|---|---|
| ΔWeight% (compared to vehicle group after 22 days) | -15% | -21% | -31% |

Conclusion: The polypeptides of the present disclosure exhibit excellent weight loss efficacy in DIO mice.

### Experimental example 6: Efficacy study in db/db mice-in vivo efficacy evaluation

### A. Experimental purpose

To study the effect of the polypeptides of the present disclosure on glycemic control in type II diabetic db/db mice.

### B. Experimental operations

1. After the db/db mice arrived at the facility, they were housed in an animal breeding room with strictly controlled environmental conditions. The temperature of the breeding room was maintained between 20 and 24°C, and the humidity was maintained between 30 and 70%. The temperature and humidity in the breeding room were monitored in real time with a thermohygrometer, and the temperature and humidity were recorded twice a day (once in the morning and once in the afternoon). The lighting in the animal breeding room was controlled by an electronic timed lighting system. The lights were turned on for 12 hours and turned off for 12 hours every day (on at 7:00 am and off at 19:00 pm). During the experiment, the animals were housed individually in cages, and toys were provided in each cage. The animals had free access to food (growth/breeding feed for rats and mice) and water during the experiment.
2. Each group of animals was subcutaneously injected with vehicle (20 mM sodium citrate buffer, pH = 7.0) and polypeptide of the present disclosure (15 nmol/kg). The administration was done between 9:30 a.m. and 11:00 a.m., once daily, for a continuous period of 4 weeks.

### C. Experimental results

The experimental results are shown in Table 11:

**Table 11 Hypoglycemic effect of the polypeptides of the present disclosure in db/db mice**

| **Polypeptide No.** | **WX001** | **WX002** | **WX005** |
|---|---|---|---|
| ΔHbA1c% (compared to vehicle group after four weeks of continuous administration) | -38.9% | -40% | -46.3% |

Conclusion: The polypeptides of the present disclosure exhibit excellent hypoglycemic efficacy in db/db mice.

## Claims

1. A polypeptide with a sequence shown in following formula or a pharmaceutically acceptable salt thereof,
Tyr-Aib-Gln-Gly-Thr-Phe-Thr-Ser-Asp-Tyr-Ser-Ile-X₁-Leu-Asp-Lys-¹Lys-Ala-Gln-¹Lys-Ala-Phe-Ile-Glu-Tyr-Leu-Leu-Glu-Gly-Gly-Pro-Ser-Ser-Gly-Ala-Pro-Pro-Pro-X₂ (I-1),
Tyr-Aib-Gln-Gly-Thr-Phe-Thr-Ser-Asp-Tyr-Ser-Ile-X₁-Leu-Asp-¹Lys-Lys-Ala-¹Lys-Aib-Ala-Phe-Ile-Glu-Tyr-Leu-Leu-Glu-Gly-Gly-Pro-Ser-Ser-Gly-Ala-Pro-Pro-Pro-X₂ (I-2),
Tyr-Aib-Gln-Gly-Thr-Phe-Thr-Ser-Asp-Tyr-Ser-Ile-X₁-Leu-Asp-Lys-Lys-Ala-Gln-¹Lys-Ala-Phe-¹Lys-Glu-Tyr-Leu-Leu-Glu-Gly-Gly-Pro-Ser-Ser-Gly-Ala-Pro-Pro-Pro-X₂ (I-3),
Tyr-Aib-Gln-Gly-Thr-Phe-Thr-Ser-Asp-Tyr-Ser-Ile-X₁-Leu-Asp-Lys-Lys-Ala-Gln-Aib-¹Lys-Phe-Ile-¹Lys-Tyr-Leu-Leu-Glu-Gly-Gly-Pro-Ser-Ser-Gly-Ala-Pro-Pro-Pro-X₂ (I-4),
Tyr-Aib-Gln-Gly-Thr-Phe-Thr-Ser-Asp-Tyr-Ser-Ile-X₁-Leu-Asp-Lys-Lys-Ala-Gln-Aib-Ala-Phe-Ile-Glu-¹Lys-Leu-Leu-¹Lys-Gly-Gly-Pro-Ser-Ser-Gly-Ala-Pro-Pro-Pro-X₂ (I-5),
Tyr-Aib-Gln-Gly-Thr-Phe-Thr-Ser-Asp-Tyr-Ser-Ile-X₁-Leu-Asp-Lys-Lys-Ala-Gln-Aib-Ala-Phe-Ile-¹Lys-Tyr-Leu-¹Lys-Glu-Gly-Gly-Pro-Ser-Ser-Gly-Ala-Pro-Pro-Pro-X₂ (II-6), wherein
Aib is 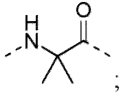
X₁ is 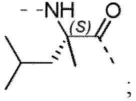
X₂ is selected from 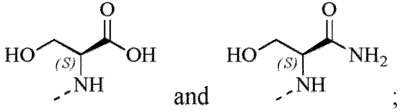
¹Lys represents a lysine with a modification, wherein the modification is that amino groups on side chains of two lysines are connected to 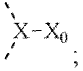
X is selected from 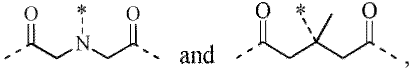 wherein "*" indicates a position connected to X₀;
X₀ is 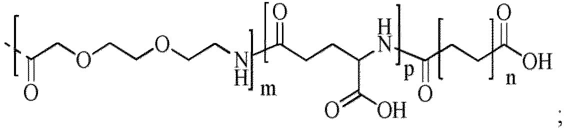
m is selected from 2 and 3;
n is selected from 8, 9, and 10;
p is selected from 1 and 2.

2. The polypeptide according to claim 1, wherein m is 2.

3. The polypeptide according to claim 1, wherein n is 9.

4. The polypeptide according to claim 1, wherein p is 1.

5. The polypeptide according to claim 1, wherein X₂ is

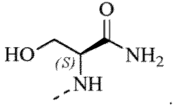

6. The polypeptide according to claim 1, wherein X₀ is

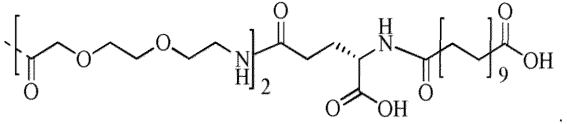

7. The polypeptide according to claim 1, wherein 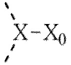 is

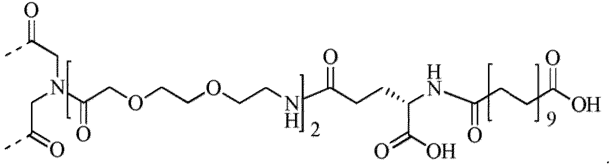

8. A polypeptide shown in following formula or a pharmaceutically acceptable salt thereof,

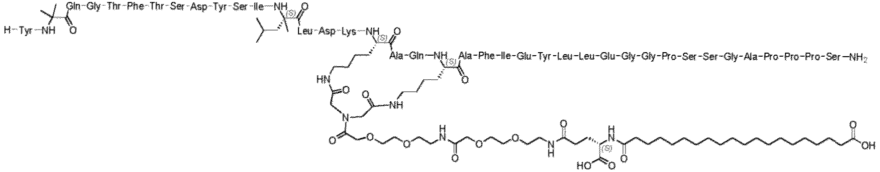

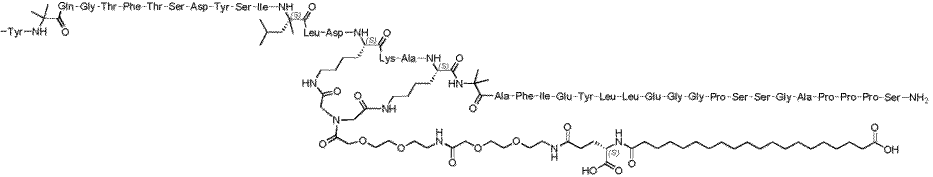

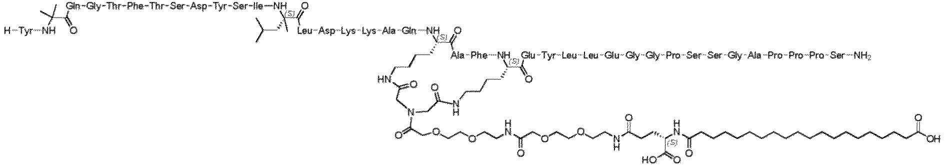

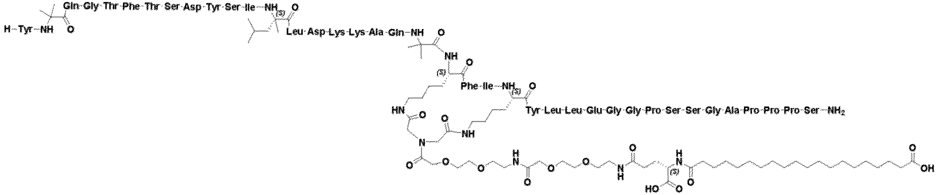

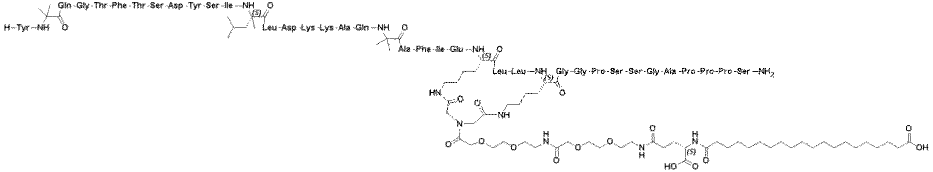

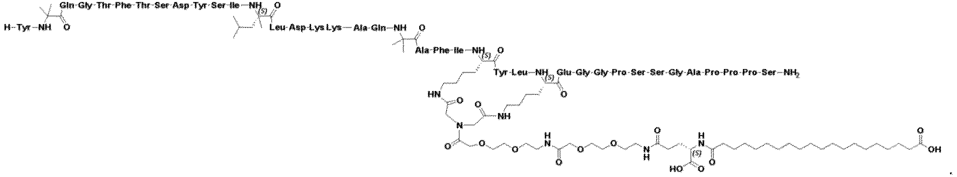

9. A pharmaceutical composition comprising a therapeutically effective amount of the polypeptide as an active ingredient or the pharmaceutically acceptable salt thereof according to any one of claims 1 to 8, or a pharmaceutically acceptable carrier.

10. A use of the polypeptide or the pharmaceutically acceptable salt thereof according to any one of claims 1 to 8, or the pharmaceutical composition according to claim 9 in the preparation of a medicament for treating diabetes and/or obesity.
